# EUROPEAN PATENT SPECIFICATION

(11) **EP 0 363 654 B1**
(45) Date of publication and mention of the grant of the patent: **18.08.1993**
(21) Application number: 89116652.2
(22) Date of filing: 08.09.1989
(51) Int. Cl.: A61F 2/60

(54) **Prosthesis**
Prothese
Prothèse

(30) Priority: 10.10.1988 FI 884462
(43) Date of publication of application: 18.04.1990
(73) Proprietor: Filppula, Martti J.O., FI-64700Teuva (FI)
(72) Inventor: Filppula, Martti J.O., FI-64700Teuva (FI)
(74) Representative: Henkel, Feiler, Hänzel & Partner

(56) References cited:
- WO-A-84/00881
- DE-A- 2 729 800
- DE-A- 3 508 919

## Description

The invention concerns a prosthesis.

Persons who have lost a limb, e.g. a leg or an arm, have to use an artificial limb or prosthesis. Such a prosthesis is usually so constructed that its shape corresponds to that of a real limb, and it is fixed in place by a technique that makes use of the part of the limb which remains after amputation, i.e. the stub.

The attachment is generally so implemented that the stub of the limb is inserted into a seat which is so shaped that it fits the form of the stub, and the prosthesis is held in place e.g. by straps or equivalent binding means, with which the prosthesis is tied to the user's body. An example of a prosthesis of the prior art is given in DE-A-3 508 919.

It is characteristic of such a prosthesis that the part of the human body which comes into direct contact with the prosthesis, i.e. the stub of the limb, and the stub seat are in close contact over a large area. A characteristic of this type of contact is that the skin very soon starts to perspire, which, besides being unpleasant for the user, is also detrimental, because in these conditions the skin easily gets into a state of irritation and becomes subject to chafing and infections, providing a fertile soil for skin diseases. In an attempt to solve this problem, talcum powder has been used between the stub and the seat, but this has not eliminated the drawbacks of the basic solution.

In many cases a person with an amputated limb must continuously wear a prosthesis every day. Thus it is obvious that the above-mentioned properties of an ordinary prosthesis make the prosthesis unpleasant to wear and may also cause health problems to the user.

The prosthesis of the invention makes it possible to achieve a decisive improvement regarding the draw-backs referred to. To implement this, the prosthesis of the invention comprises a seat which receives the stub of the limb when the prosthesis is in use and a means allowing ventilation at the boundary surface between the prosthesis and the stub of the limb, wherein the ventilating means comprises the seat which permits a ventilating medium, preferably air, to flow through it, one or more sources of ventilating medium and a connecting channel between the seat and the source.

The chief advantage provided by the invention is that it offers the user more pleasant conditions for wearing the prosthesis, the prosthesis can be worn continuously for a longer time than before, and no skin irritation occurs.

In the following, the invention is described in detail with reference to the attached drawing, in which
Fig.1 presents an artificial limb representing the state of the art.
Fig. 2 presents a sectional view of a prosthesis constructed as provided by the invention.
Fig. 3 presents a sectional view of another prosthesis constructed as provided by the invention.

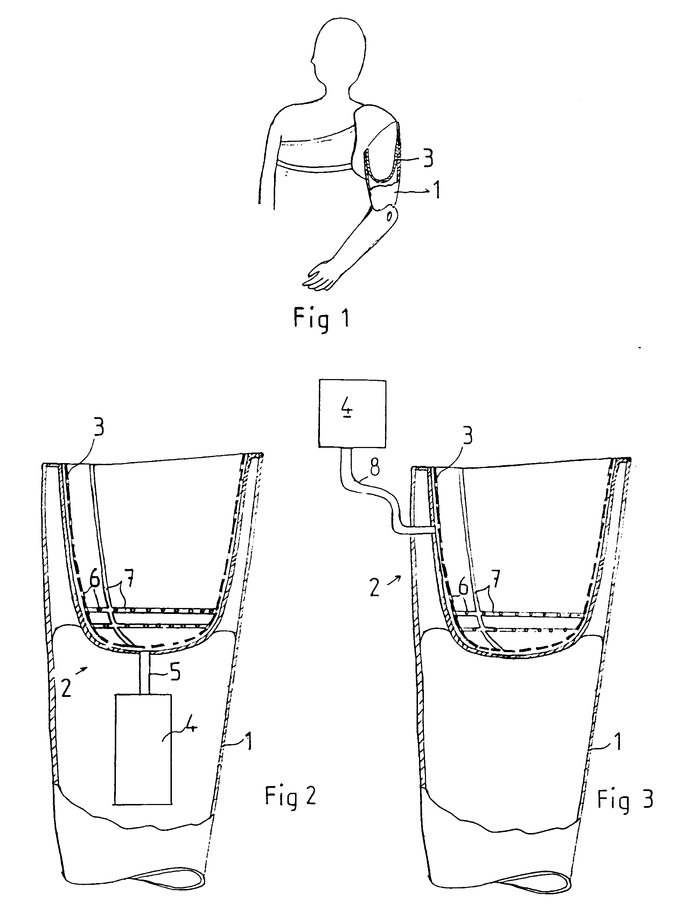

Fig. 1 shows a typical example of a prosthesis representing the state of the art, designed for a limb, in this case arm. The prosthesis, shown in a partially sectioned view in Fig. 1, is directly suspended on the remaining part of the arm, with the stub of the arm inserted into a seat provided within the prosthesis and shaped so as to fit the form of the stub, a preferable form of the seat being that of a sheathlike sleeve 3. With such a suspension arrangement, an artificial limb is achieved that feels as natural as possible and also permits of limited motion similar to that of a normal arm.

Fig. 2 shows a sectional view of that part of a prosthesis constructed as provided by the invention which accommodates the stub of the limb. The means 2 of the invention for ventilating the boundary surface between the stub of the limb and the prosthesis comprises a seat 3 which permits a ventilating medium, preferably air, to pass through it, and which is shaped to fit the form of the stub of the limb and receives the stub when the prosthesis is in use. The ventilating means also comprises a source 4 of ventilating medium which either contains or produces a ventilating medium, and a connecting channel 5 between the seat 3 and the source 4. The seat 3 is preferably of the form of a sheathlike sleeve, corresponding to the form of the stub of the limb, and it is provided with apertures 6 and 7, preferably consisting of holes and channels on the surface of the seat. These apertures, holes and channels serve to help the ventilating medium flow out of the prosthesis. The sleeve 3 can also be made of a porous material permitting the ventilating medium to flow through it. The source 4 of ventilating medium may preferably consist of a pressure reservoir containing compressed air, preferably of a construction permitting detachment and replacement. The source 4 may also consist of a pump, an air impeller or an equivalent device producing compressed air or a flow of air, and it can preferably be placed inside the prosthesis as shown in Fig. 2.

When the ventilating means is to be operated, the user activates the source 4 of ventilating medium, so that the ventilating medium will flow through the connecting channel 5, 8 into the seat 3, preferably shaped in the form of a sheathlike sleeve and provided with apertures 6 and/or channels 7. The schematic diagram in Fig. 2 shows only a few of such apertures and channels, but it is obvious that there can be as many of them as is desirable in view of the effect aimed at.

Fig. 3 shows a sectional view of a prosthesis corresponding to that in Fig. 2, with the difference that in this embodiment the source 4 of ventilating medium is placed outside the prosthesis. In this case the ventilating medium, preferably air, is supplied into the seat 3 through channel 8. Placing the source of ventilating medium outside the prosthesis may provide an advantage in the case of a prosthesis designed for the lower limbs, in which case the device (not shown) activating the source 4 of ventilating medium can be more easily operated by the user.

As stated above, the source 4 of ventilating medium may be a pump, an impeller or an equivalent air flow generator, and it may be driven by an electric motor powered by a rechargeable accumulator or battery.

In a preferred embodiment of of the invention, the prosthesis is provided with a filter, mounted between the source of ventilating medium and the seat or sleeve, e.g. in the connecting channel between them, the filter serving to remove detrimental particles from the ventilating medium. The filter may naturally be of any type.

In another preferred embodiment of the invention, the prosthesis is provided with a device for heating the ventilating medium, said device being preferably controlled by a thermostat in such manner that the temperature of the ventilating medium is kept at a level corresponding to the body temperature, so that the air reaching the limb seat in the prosthesis feels pleasant to the user.

The invention is not restricted to the examples of its embodiments described above, but many variations are possible within the scope of the idea of the invention as defined in the claims.

## Claims

1. A prosthesis (1) which comprises a seat (3) which receives the stub of the limb when the prosthesis is in use and a means (2) for ventilating the boundary surface between the stub of the limb and the prosthesis **characterized** in that the ventilating means (2) comprises the seat (3) which permits a ventilating medium, preferably air, to flow through it, one or more sources (4) of ventilating medium and a connecting channel (5) between the seat (3) and the source (4).

2. Prosthesis according to claim 1, **characterized** in that the seat (3) is provided with apertures (6, 7) providing an outlet for the ventilating medium, or the seat (3) is made of a porous material.

3. Prosthesis according to any one of claims 1 and 2, **characterized** in that the source (4) of ventilating medium is a pressure reservoir, which is preferably so constructed that it can be detached and replaced.

4. Prosthesis according to any one of claims 1 and 2, **characterized** in that the source (4) of ventilating medium is a pump.

5. Prosthesis according to any one of claims 1 - 4, **characterized** in that the source (4) of ventilating medium is located inside the prosthesis.

6. Prosthesis according to any one of claims 1 - 4, **characterized** in that the source (4) of ventilating medium is located outside the prosthesis, and that the means comprises a connecting channel (8) between the seat (3) and the source (4).

7. Prosthesis according to any one of claims 4 - 6, **characterized** in that the pump (5) is driven by an electric motor.

8. Prosthesis according to claim 7, **characterized** in that the electric motor is powered by a rechargeable accumulator or battery.

9. Prosthesis according to claim 1 - 8, **characterized** in that the ventilating means (2) comprises a filter for purifying the ventilating medium.

10. Prosthesis according to claim 1 - 9, **characterized** in that the ventilating means (2) comprises a heating device for heating the ventilating medium.

## Patentansprüche

1. Prothese (1) mit einem Sitz (3), der im Gebrauch der Prothese den Gliedstumpf aufnimmt, und einer Einrichtung (2) zum Belüften oder Ventilieren der Grenzfläche zwischen dem Gliedstumpf und der Prothese, dadurch gekennzeichnet, daß die Belüftungseinrichtung (2) den Sitz (3), der ein Belüftungsmedium, vorzugsweise Luft, hindurchströmen läßt, eine oder mehrere Quellen (4) für Belüftungsmedium und einen Verbindungskanal (5) zwischen dem Sitz (3) und der Quelle (4) umfaßt.

2. Prothese nach Anspruch 1, dadurch gekennzeichnet, daß der Sitz (3) mit Öffnungen (6, 7) zur Bereitstellung eines Auslasses für das Belüftungsmedium versehen oder der Sitz (3) aus einem porösen Werkstoff geformt ist.

3. Prothese nach einem der Ansprüche 1 und 2, dadurch gekennzeichnet, daß die Quelle (4) für das Belüftungsmedium ein Druck(vorrats)behälter ist, der vorzugsweise so ausgebildet ist, daß er abgenommen und ausgewechselt werden kann.

4. Prothese nach einem der Ansprüche 1 und 2, dadurch gekennzeichnet, daß die Quelle (4) für Belüftungsmedium eine Pumpe ist.

5. Prothese nach einem der Ansprüche 1 bis 4, dadurch gekennzeichnet, daß die Quelle (4) für Belüftungsmedium innerhalb der Prothese angeordnet ist.

6. Prothese nach einem der Ansprüche 1 bis 4, dadurch gekennzeichnet, daß die Quelle (4) für Belüftungsmedium außerhalb der Prothese angeordnet ist und daß die Einrichtung einen Verbindungskanal (8) zwischen dem Sitz (3) und der Quelle (4) aufweist.

7. Prothese nach einem der Ansprüche 1 bis 6, dadurch gekennzeichnet, daß die Pumpe (5 bzw. 4) durch einen Elektromotor antreibbar ist.

8. Prothese nach Anspruch 7, dadurch gekennzeichnet, daß der Elektromotor durch eine(n) wiederaufladbare(n) Akkumulator oder Batterie speisbar ist.

9. Prothese nach den Ansprüchen 1 bis 8, dadurch gekennzeichnet, daß die Belüftungseinrichtung (2) ein Filter zum Reinigen des Belüftungsmediums aufweist.

10. Prothese nach den Ansprüchen 1 bis 9, dadurch gekennzeichnet, daß die Belüftungseinrichtung (2) eine Erwärmungsvorrichtung zum Erwärmen des Belüftungsmediums aufweist.

## Revendications

1. Prothèse (1) comprenant un siège (3) recevant le moignon du membre lorsque la prothèse est utilisée et un moyen (2) pour aérer la surface mitoyenne entre la moignon du membre et la prothèse, caractérisé en ce que la moyen d'aération (2) comprend le siège (3) qui permet à une substance d'aération, de préférence de l'air, de s'écouler à travers la prothèse, une ou plusieurs sources (4) de substance d'aération et un canal de raccordement (5) entre le siège (3) et la source (4).

2. Prothèse selon la revendication 1, caractérisée en ce que le siège (3) est équipé d'ouvertures (6, 7) fournissant une sortie pour la substance d'aération, ou en ce que le siège (3) est construit en matériau poreux.

3. Prothèse selon l'une quelconque des revendications 1 et 2, caractérisée en ce que la source (4) de la substance d'aération est un réservoir à pression do préférence construit do telle sorte qu'il puisse être détaché et remplacé.

4. Prothèse selon l'une quelconque des revendications 1 at 2, caractérisée en ce que la source (4) de la substance d'aération est une pompe.

5. Prothèse selon l'une quelconque des revendications 1 à 4, caractérisée en ce que il source (4) de la substance d'aération est située à l'intérieur de la prothèse.

6. Prothèse selon l'une quelconque des revendications 1 à 4, caractérisée en ce que la source (4) de la substance d'aération est située à l'extérieur de la prothèse, et que le moyen comprend un oanal de raccordement (6) entre le siège (3) et la source (4).

7. Prothèse selon l'une quelconque des revendications 4 à 6, caractérisée en ce que le pompe (5) est commandée par un moteur électrique.

8. Prothèse selon la revendication 7, caractérisée en ce que le moteur électrique est alimenté par un accumulateur ou une batterie rechargeable.

9. Prothèse selon les revendications 1 à 8, caractérisée en ce que le moyen de ventilation (2) comprend un filtre pour purifier la substance d'aération.

10. Prothèse selon les revendications 1 à 9, caractérisée en ce que le moyen de ventilation (2) comprend un dispositif de chauffage pour chauffer la substance d'aération.
